(19) Europäisches Patentamt
European Patent Office
Office européen des brevets

(11) Veröffentlichungsnummer: **0 254 224**
**A2**

# (12) EUROPÄISCHE PATENTANMELDUNG

(21) Anmeldenummer: **87110365.1**

(22) Anmeldetag: **17.07.87**

(51) Int. Cl.⁴: **C07D 219/08**, C07D 401/04, A61K 31/435

(30) Priorität: **22.07.86 DE 3624702**

(43) Veröffentlichungstag der Anmeldung:
**27.01.88 Patentblatt 88/04**

(84) Benannte Vertragsstaaten:
**AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Dhar, Rajkumar, Dr.**
**G-1, Hoechst Quarters Mulund (West)**
**Bombay 400 082(IN)**
Erfinder: **Venugopalan, Bindumadhavan, Dr.**
**C, 106, Usha Nagar Bhandup**
**Bombay 400 078(IN)**
Erfinder: **Chatterjee, Dipak Kumar, Dr.**
**G-21, Hoechst Quarters Mulund (West)**
**Bombay 400 082(IN)**
Erfinder: **Rupp, Richard Helmut, Dr.**
**Niladri 66 Nepean Sea Road**
**Bombay 400 006(IN)**
Erfinder: **de Souza, Noel John, Dr.**
**Melrose 62 Pali Hill**
**Bandra Bombay 400 050(IN)**

(54) **1-substituierte 3-Aryl-7-chlor-3,4-dihydro(2H)-acridon-N-oxide, ein Verfahren zu ihrer Herstellung und ihre Verwendung als Arzneimittel und Futtermittelzusatz.**

(57) Die vorliegende Erfindung betrifft neue, 1-substituierte 3-Aryl-7-chlor-3,4-dihydro-(2H)-aridon-N-oxide, ein Verfahren zu deren Herstellung, diese enthaltende pharmazeutische Zusammensetzungen sowie ihre Verwendung als chemotherapeutische Mittel, insbesondere als Malariamittel und Coccidiostatika.

**EP 0 254 224 A2**

Xerox Copy Centre

# 1-substituierte 3-Aryl-7-chlor-3,4-dihydro(2H)-acridon-N-oxide, ein Verfahren zu ihrer Herstellung und ihre Verwendung als Arzneimittel und Futtermittelzusatz

Die vorliegende Erfindung betrifft neue, 1-substituierte 3-Aryl-7-chlor-3,4-dihydro-(2H)-acridon-N-oxide, ein Verfahren zu deren Herstellung, diese enthaltende pharmazeutische Zusammensetzungen sowie ihre Verwendung als chemotherapeutische Mittel, insbesondere als Malariamittel und Coccidiostatika.

Die folgenden Patente und Veröffentlichungen betreffen 3-Aryl-7-chlor-3,4-dihydro-10-hydroxy-1,9-(2H,1OH)-acridindion und dessen Derivate:
Deutsche Patentschrift 23 37 474
Deutsche Offenlegungsschrift 27 48 333
Arzneimittelforschung, Drug Research 30(11), Nr. 7, S. 1041-46 (1980)
Eur. J. Med. Chem., CHIM. THER. 20 Nr. 4, S. 363-370 (1985).

Einige der beschriebenen Acridone besitzen eine chemotherapeutische Wirkung gegen Protozoeninfektionen.

Die erfindungsgemäßen Verbindungen, sind bisher nicht in der Literatur beschrieben. Sie unterscheiden sich in ihrer Konstitution von den aus dem Stand der Technik bekannten Verbindungen dadurch, daß sie N-Oxide des Acridonmoleküls darstellen und in 1-Stellung Alkoxy-bzw. substituierte Aminosubstituenten tragen. Die erfindungsgemäßen Verbindungen sind äußerst wirksam gegen Malaria-und Coccidienparasiten.

Da Malariaparasiten rasch eine Resistenz gegen die üblicherweise verwendeten Arzneimittel ausbilden, besteht ein Bedarf an neuen sicheren und wirksamen Malariamitteln.

Die vorliegende Erfindung ist ein Ergebnis der Bemühungen neue Verbindungen mit hoher Wirksamkeit gegen Malaria ohne unerwünschte Nebenwirkungen zu entwickeln.

Gegenstand der vorliegenden Erfindung sind daher 1-substituierte 3-Aryl-7-chlor-3,4-dihydro-2(H)-acridon-N-oxide der Formel I

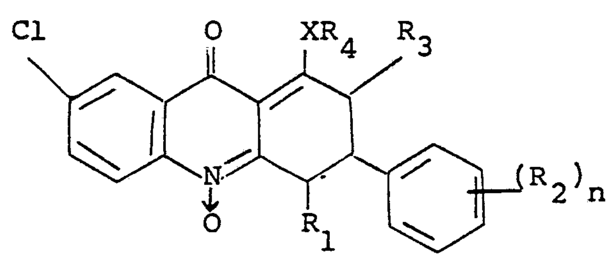

I

worin $R_1$ und $R_3$ gleich oder verschieden sind und für Wasserstoff, $C_1$-$C_4$-Alkyl oder eine $C_2$-$C_5$-Carbalkoxy-oder Arylgruppe steht, $R_2$ Halogen oder Trifluormethyl bedeutet, wobei die Substituenten, falls n eine Zahl von 2 oder 3 bedeutet, gleich oder verschieden sein können, n für eine Zahl von 0 bis 3, X für Sauerstoff oder Stickstoff und $R_4$, falls X Sauerstoff bedeutet, für eine geradkettige oder verzweigte $C_1$-$C_4$-Alkylgruppe, oder falls X Stickstoff bedeutet, $XR_4$ für eine Di-$C_1$-$C_4$-alkylaminogruppe oder einen fünf-oder sechsgliedrigen Heterocyclus, der ein weiteres Heteroatom enthalten kann und gegebenentalls ein-oder mehrfach mit $C_1$-$C_4$-Alkyl, substituierter $C_1$-$C_4$-Alkyl oder einer gegebenenfalls einfach oder mehrfach mit $C_1$-$C_4$-Alkyl, $C_1$-$C_4$-Alkoxy oder Halogen substituierten Arylgruppe substituiert ist, stehen.

Wenn $R_1$ bzw $R_4$ eine $C_1$-$C_4$-Alkylgruppe bedeuten, so handelt es sich um eine Methyl-, Äthyl-, Propyl-, Isopropyl-oder Butylgruppe.

Steht $R_2$ für Halogen, so bedeutet es Chlor, Brom, Fluor oder Jod.

$R_1$ und $R_3$ als $C_2$-$C_5$-Carbalkoxygruppe bedeuten bevorzugt Carbomethoxy oder Carboäthoxy.

Bei einere Dialkylaminogruppe $XR_4$ handelt es sich bevorzugt um eine Dimethylamino-oder Diäthylaminogruppe.

Unter $C_1$-$C_4$-Alkoxy ist bevorzugt die Methoxy-oder Äthoxygruppe zu verstehen.

Steht $XR_4$ für einen fünf-oder sechs-gliedrigen Heterocyclus, so sind dies bevorzugt Heterocyclen wie Pyrrolidino, Piperidino, Morpholino oder Piperazino, die gegebenenfalls substituiert sind mit $C_1$-$C_4$-Alkyl-oder eine Arylgruppe, welche ein-oder mehrfach substituiert sein kann, gegebenenfalls mit $C_1$-$C_4$-Alkyl, Halogen, $C_1$-$C_4$-Alkoxy oder Trifluormethyl. Alkyl, Halogen und Alkoxy haben dabei dieselbe Bedeutung wie oben angegeben.

Unter Aryl ist bevorzugt Phenyl zu verstehen.

Verbindungen der Formel I, in denen $R_1$ für Wasserstoff, $R_3$ für Wasserstoff oder Methyl, $R_2$ für Chlor oder eine Trifluormethylgruppe, X für Sauerstoff oder Stickstoff und $R_4$ für Methyl, Äthyl oder Isopropyl bzw. $XR_4$ für einen gegebenenfalls substituierten Piperazino-, Pioeridino-oder Pyrrolidinorest stehen, und n = 1 ist, sind bevorzugt.

Besonders bevorzugte erfindungsgemäße Verbindungen sind:

7-Chlor-3,4-dihydro-1-methoxy-3-(4-trifluormethylphenyl)-2(H)-acridon-N-oxid-monohydrat,
7-Chlor-3,4-dihydro-1-äthoxy-3-(4-trifluormethylphenyl)-2(H)-acridon-N-oxid-sesquihydrat,
7-Chlor-3,4-dihydro-1-isopropoxy-3-(4-trifluormethylphenyl)-2(H)-acridon-N-oxid,
7-Chlor-3,4-dihydro-1-äthoxy-2-methyl-3-(4-trifluormethylphenyl)-2(H)-acridon-N-oxid-dihydrat,
7-Chlor-3,4-dihydro-2-methyl-1-(N-methylpiperazino)-3-(4-trifluormethylphenyl)-2(H)-acridon-N-oxid-monohydrat,
7-Chlor-3,4-dihydro-1-pyrrolidino-3-(4-trifluormethylphenyl)-2(H)-acridon-N-oxid-monohydrat,
7-Chlor-3,4-dihydro-1-(N-2,6-dimethylphenylpiperazino)-3-(4-trifluormethylphenyl)-2(H)-acridon-N-oxid-monohydrat,
7-Chlor-3,4-dihydro-1-(N-4-methoxyphenylpiperazino)-3-(4-trifluormethylphenyl)-2(H)-acridon-N-oxid-monohydrat,
7-Chlor-3,4-dihydro-1-[N-(N'-benzylpiperazino)]-3-(4-trifluormethylphenyl)-2(H)-acridon-N-oxid-monohydrat,
7-Chlor-3,4-dihydro-1-(N-phenylpiperazino)-3-(4-trifluormethylphenyl)-2(H)-acridon-N-oxid-monohydrat,
7-Chlor-3,4-dihydro-1-(N-3-trifluormethylphenylpiperazino)-3-(4-trifluormethylphenyl)2(H)-acridon-N-oxid-monohydrat,
7-Chlor-3,4-dihydro-1-morpholino-3-(4-trifluormethylphenyl)-2(H)-acridon-N-oxid-monohydrat,
7-Chlor-3,4-dihydro-1-[N-2-methoxyphenylpiperazino]-3-(4-chlorphenyl)-2(H)-acridon-N-oxid,
7-Chlor-3,4-dihydro-1-(N-methylpiperazin)-3-(4-trifluormethylphenyl)2(H)-acridon-N-oxid-monohydrat,
7-Chlor-3,4-dihydro-1-[N-(2-methyl-N'-phenylpiperazino)]-3-(4-trifluormethylphenyl)-2(H)-acridon-N-oxid-monohydrat,
7-Chlor-3,4-dihydro-1-[N-(N'-2-methylphenylpiperazino)]-3-(4-trifluormethylphenyl)-2(H)-acridon-N-oxid-monohydrat und 7-Chlor-3,4-dihydro-1-[(N-4-(p-chlorphenyl)-3-methylpiperazino]-3-(4-trifluormethylphenyl)-(H2)-acridon-N-oxid-monohydrat.

Einige der neuen, erfindungsgemäßen N-Oxide sind in der nachfolgenden Tabelle I aufgeführt.

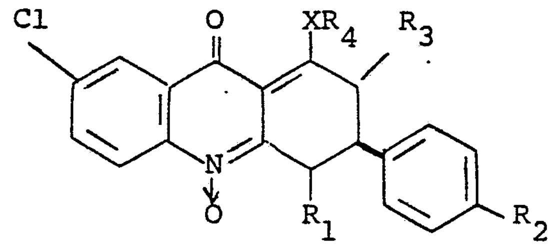

| $R_1$ | $R_2$ | $R_3$ | $XR_4$ | Y | Schmelzpunkt |
|---|---|---|---|---|---|
| H | Cl | H | 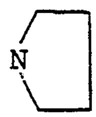 | - | > 300 |
| H | Cl | H | 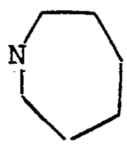 | - | 222-23 |
| H | Cl | H | 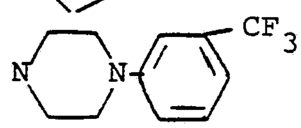 | $H_2O$ | 197-200 |
| H | Cl | H | 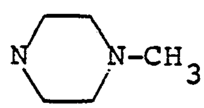 | - | 220-22 |
| H | Cl | H | 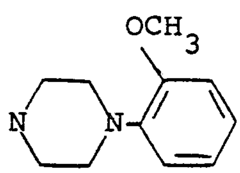 | - | 188-89 |
| H | Cl | H | 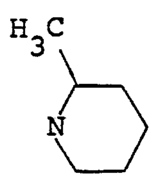 | $1,5H_2O$ | 200-201 |
| H | Cl | H | 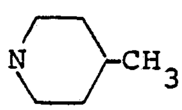 | $H_2O$ | 215-216 |
| H | Cl | H | 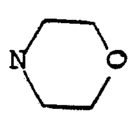 | $H_2O$ | 250 (Zers.) |

| $R_1$ | $R_2$ | $R_3$ | $XR_4$ | Y | Schmelzpunkt |
|---|---|---|---|---|---|
| H | $CF_3$ | H | 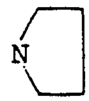 | $H_2O$ | 215 (Zers.) |
| H | $CF_3$ | H | 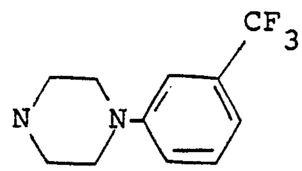 | $H_2O$ | 210-11 |
| H | $CF_3$ | H | $N(CH_3)_2$ | $H_2O$ | 230 |
| H | $CF_3$ | H | 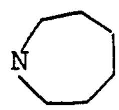 | $1,5H_2O$ | 220-25 |
| H | $CF_3$ | H | 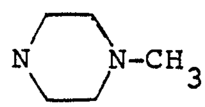 | $H_2O$ | 215-220 |
| H | $CF_3$ | H | 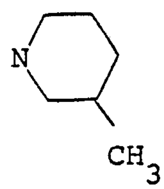 | $H_2O$ | 205-205 |
| H | $CF_3$ | H | 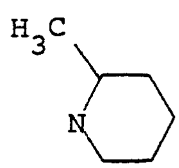 | $H_2O$ | 205-206 |
| H | $CF_3$ | H | 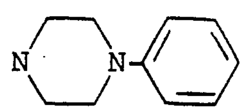 | $H_2O$ | 197-98 |
| H | $CF_3$ | H | 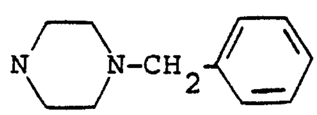 | $H_2O$ | 199-200 |
| H | $CF_3$ | H | 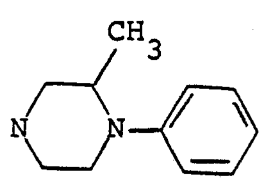 | $H_2O$ | 209-210 |

| $R_1$ | $R_2$ | $R_3$ | $XR_4$ | Y | Schmelzpunkt |
|---|---|---|---|---|---|
| H | $CF_3$ | H | 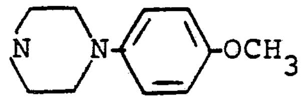 | $H_2O$ | 204-5 |
| H | $CF_3$ | H | 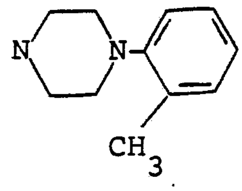 | $H_2O$ | 212-13 |
| H | $CF_3$ | H | 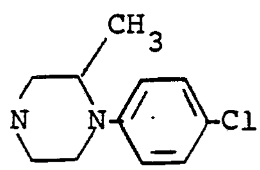 | $H_2O$ | 207-8 |
| H | $CF_3$ | H | 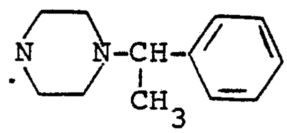 | $H_2O$ | 184-85 |
| H | $CF_3$ | H | 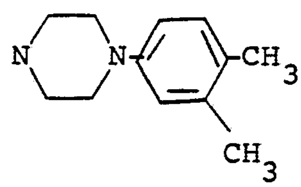 | $H_2O$ | 209-10 |
| H | $CF_3$ | H | 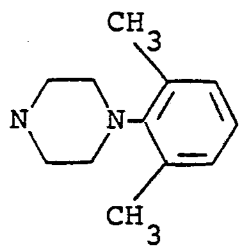 | $H_2O$ | 221-22 |
| H | $CF_3$ | H | 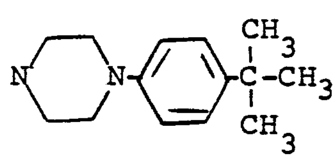 | $H_2O$ | 203-4 |
| H | $CF_3$ | H | 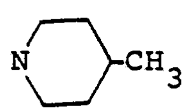 | $H_2O$ | 203-4 |

| $R_1$ | $R_2$ | $R_3$ | $XR_4$ | Y | Schmelzpunkt |
|---|---|---|---|---|---|
| H | $CF_3$ | H | 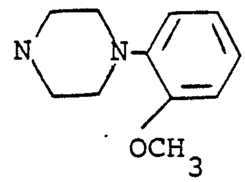 | - | 214-215 |
| H | $CF_3$ | H | 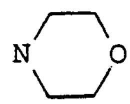 | $H_2O$ | 230-36 |
| H | $CF_3$ | $CH_3$ | 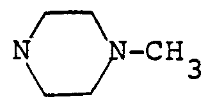 | $H_2O$ | 290-92 |
| H | $CF_3$ | H | $OCH(CH_3)_2$ | - | > 300 |
| H | $CF_3$ | H | $OCH_3$ | $H_2O$ | 292-94 |
| H | $CF_3$ | H | $OC_2H_5$ | $2.5H_2O$ | > 300 |
| H | $CF_3$ | $CH_3$ | $OC_2H_5$ | $2.H_2O$ | 296 |
| H | $CF_3$ | $C_6H_5$ | $OC_2H_5$ | - | > 300 |

Gegenstand der vorliegenden Erfindung ist ferner ein Verfahren zur Herstellung der Verbindungen der Formel I, welches dadurch gekennzeichnet ist, daß man ein entsprechend substiutiertes Acridindion der allgemeinen Formel II in einem geeigneten wasserfreien Alkohol mit entsprechenden Alkoxiden der Formel III bzw. allgemein in einem wasserfreien polaren oder unpolaren Lösungsmittel wie Methanol, Äthanol, t-Butanol, Benzol oder N,N-Dimethylformamid mit einem entsprechenden Amin der Formel IV, zweckmäßig bei einer Temperatur von Raumtemperatur bis 100°C und vorteilhaft über einen Zeitraum von 1 bis 24 Stunden zur Reaktion bringt, wie beispielsweise unten angegeben:

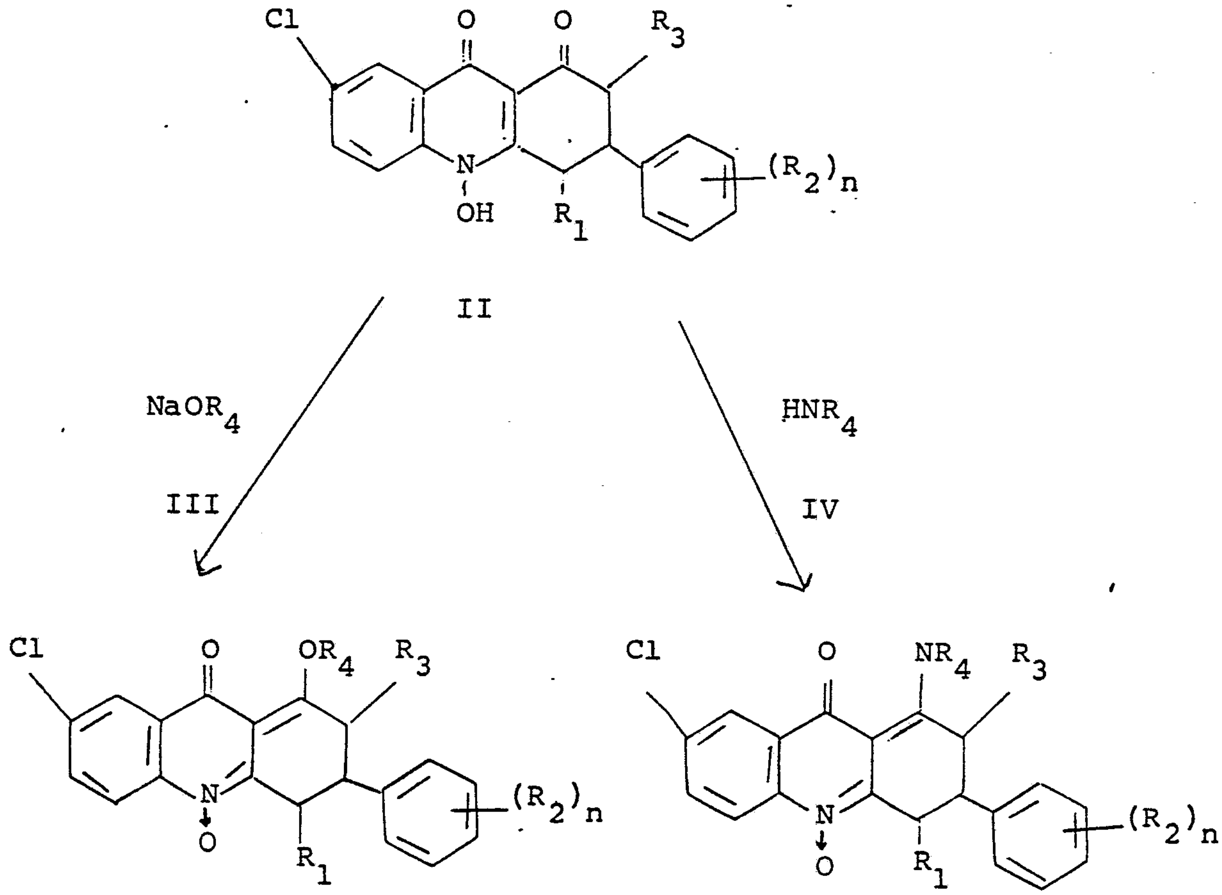


worin $R_1$ bis $R_4$ und n die oben angegebenen Bedeutungen haben. Unter bevorzugten Bedingungen wendet man alkoholische Lösungsmittel und Raumtemperatur an.

Die Ausgangsacridone der Formel (II) sind auf analoge Weise herstellbar wie in der Deutschen Patentschrift 2 337 474 und in Arzneimittelforschung, Drug Research 30 (II), Nr. 7, S. 363 - 370 (1980) beschrieben und durch das folgende Reaktionsschema I erläutert.

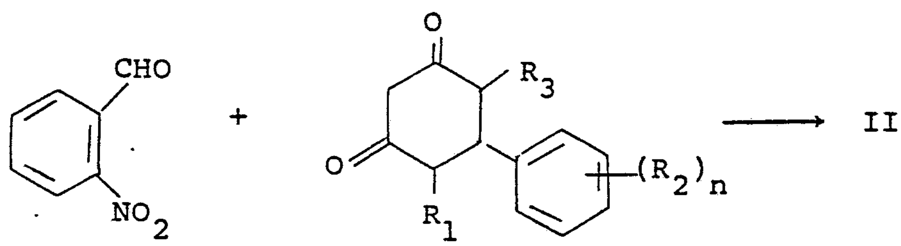


Schema I

worin $R_1$, $R_2$, $R_3$ die oben angegebenen Bedeutungen haben. Die Verbindungen (I) können aus organischen Lösungsmitteln wie Chloroform, Methanol oder Aceton umkristallisiert oder durch Anreiben in diesen gereinigt werden.

Die erfindungsgemäßen Verbindungen besitzen wertvolle chemotherapeutische, zur Bekämpfung von Protozoeninfektionen geeignete Eigenschaften. Beispielsweise zeichnen sie sich durch eine hohe Wirksamkeit gegen Plasmodien, die Malariaerreger, aus. An geeigneten Modellen konnte auch eine Wirksamkeit gegen den Chloroquin-resistenten Stamm von Plasmodium berghei gezeigt werden. Bei Verabreichung an mit Plasmodium berghei infizierte Mäuse in einer Dosis im Bereich von 10 - 25 mg/kg $\times$ 5 z.B. wird die Parasitaemie vollständig geheilt.

Die Verbindungen der Formel I kann man peroral oder parenteral in Dosierungen im Bereich von 2,5 bis 100 mg/kg Körpergewicht verabreichen. Als Anti-Malaria-Mittel werden Dosiereinheitsformen wie Dragees oder Kapseln zur oralen Verabreichung bzw. Lösungen oder Suspensionen für Injektionen mit je 100 bis 400 mg Wirkstoff bevorzugt. Solche Dosiereinheiten werden je nach dem Befinden des Patienten ein-bis dreimal täglich verabreicht.

Gegenstand vorliegender Erfindung sind ferner pharmazeutische Präparate, die eine erfindungsgemäße Verbindung im Gemisch oder zusammen mit einem pharmazeutisch geeigneten Träger enthalten. Die Präparate können in zur oralen oder parenteralen Verabreichung geeigneter Form und vorzugsweise in Dosiereinheitsformen vorliegen.

Zur oralen Verabreichung kommen Tabletten, Dragees, Kapseln, Pulver oder Granulat in Frage, welche den Wirkstoff im Gemisch oder zusammen mit beispielsweise Stärke, Cellulosepulver, Talkum, Magnesiumstearat, Zucker, Gelatine, Calciumcarbonat, feinverteilter Kieselsäure oder Carboxymethylcellulose enthalten.

Zur parenteralen Verabreichung, insbesondere für intramuskuläre Injektionen, kommen sterile Suspensionen, zum Beispiel unter Verwendung von Sesamöl, Rizinusöl oder synthetischen Triglyceriden hergestellte ölige Suspensionen, gegebenenfalls unter gleichzeitiger Anwendung von Tensiden, beispielsweise Sorbitan-Fettsäureester, in Betracht. Zudem kann man auch wäßrige Suspensionen beispielsweise unter Verwendung äthoxylierter Sorbitan-Fettsäureester, gegebenenfalls unter Zusatz von Verdickungsmitteln, beispielsweise Polyäthylenglykol oder Carboxymethylcellulose, herstellen.

Darüber hinaus sind die erfindungsgemäßen Verbindungen auch gegen Coccidioserreger wirksam, beispielsweise Geflügel-, Puten-, Kaninchen-, Rinder-und Schweinecoccidiose. Bei modernen Schlachttierzuchtmethoden stellt die Coccidiase während der Aufzuchts-und Mästzeit ein ernsthaftes Problem dar, da sie erhebliche wirtschaftliche Verluste verursachen kann. Dementsprechend besteht ein großes Interesse an der Verfügbarkeit hochaktiver, gut tolerierter Coccidiostatika.

Dazu werden die Verbindungen der Formel I im Gemisch mit geeigneten Futtermitteln verabreicht. Gegenstand der Erfindung ist demnach auch ein Futtermittelzusatz, welcher eine Verbindung der Formel I enthält.

Die nachfolgenden Beispiele erläutern die Erfindung.

Herstellungsbeispiele:

Beispiel 1:

7-Chlor-3,4-dihydro-1-pyrrolidino-3-(4-trifluormethylphenyl)-2(H)-acridon-N-oxid-monohydrat

Man rührt eine Suspension von 7-Chlor-3,4-dihydro-10-hydroxy-3-(4-trifluormethylphenyl)-1,9(2H,10H)-acridon (1,02 g, 0,0025 Mol) in Methanol (10 ml) bei Raumtemperatur und tropft Pyrrolidin (0,22 ml, 0,0032 Mol) dazu, wobei sich eine klare Lösung bildet. Bei weiterem Rühren erscheint ein gelber Niederschlag im Reaktionsgemisch, das noch eine halbe Stunde weitergeführt und dann filtriert wird. Das dabei erhaltene Produkt wird mit Aceton gewaschen und im Vakuum getrocknet, wobei man 7-Chlor-3,4-dihydro-1-pyrrolidino-3-(4-trifluormethylohenyl)-acridon-N-oxidmonohydrat vom Schmelzpunkt 215°C in 78 % Ausbeute (0,9 g) erhält.

Unter den oben beschriebenen Bedingungen und unter Einsatz eines entsprechend substituierten Acridindions und des entsprechenden Amins anstelle von Pyrrolidin werden folgende Verbindungen hergestellt:

7-Chlor-3,4-dihydro-1-(N-2,6-dimethylohenylpiperazino)-3-(4-trifluormethylphenyl)-2(H)-acridon-N-oxid-monohydrat, Schmelzpunkt 221-22°C;
7-Chlor-3,4-dihydro-1-(N-4-methoxyphenylpiperazino)-3-(4-trifluormethylphenyl)-2(H)-acridon-N-oxid-monohydrat, Schmelzpunkt 204-5°C;
7-Chlor-3,4-dihydro-1-[N-(N-benzylpiperazino)]-3-(4-trifluormethylphenyl)-2(H)-acridon-N-oxid-monohydrat; 7-Chlor-3,4-dihydro-1-(N-phenylpiperazino)-3-(4-trifluormethylphenyl)-2(H)-acridon-N-oxid-monohydrat, Schmelzpunkt 197-98°C;
7-Chlor-3,4-dihydro-1-(N-3-trifluormethylphenylpiperazino)-3-(4-trifluormethylphenyl)-2(H)-acridon-N-oxid-monohydrat, Schmelzpunkt 210-11°C;
7-Chlor-3,4-dihydro-1-(morpholino)-3-(4-trifluormethylphenyl)-2(H)-acridon-N-oxid-monohydrat, Schmelzpunkt 250°C (Zers.);

7-Chlor-3,4-dihydro-1-[N-2-methoxyphenylpiperazino]-3-(4-chlorphenyl)-2(H)-acridon-N-oxid, Schmelzpunkt 188-89°C;
7-Chlor-3,4-dihydro-1-(N-methylpiperazino)-3-(4-trifluormethylphenyl)-2(H)-acridon-N-oxid-monohydrat, Schmelzpunkt 215-20°C;
7-Chlor-3,4-dihydro-1-[N-2-methyl-N'-phenylpiperazino]-3-(4-trifluormethylphenyl)-2(H)-acridon-N-oxid-monohydrat, Schmelzpunkt 209-210°C;
7-Chlor-3,4-dihydro-1-[N-4-(p-chlorphenyl)-3-methylpiperazino]-3-(4-trifluormethylphenyl)-2(H)-acridon-N-oxid-monohydrat, Schmelzpunkt 207-208°C;
7-Chlor-3,4-dihydro-2-methyl-1-(N-methylpiperazino)-3-(4-trifluormethylphenyl)-2(H)-acridon-N-oxid-monohydrat, Schmelzpunkt 290-92°C.

Beispiel 2:

7-Chlor-3,4-dihydro-1-methoxy-3-(4-trifluormethylphenyl)-2(H)-acridon-N-oxid-monohydrat

Unter gutem Rühren versetzt man eine durch Auflösen von Natrium (1,2 g) in wasserfreiem Methanol (100 ml) hergestellte Natriummethylatlösung mit 7-Chlor-3,4-dihydro-10-hydroxy-3-(4-trifluormethylphenyl)-1,9(2H,10H)-acridindion (10,0 g), rührt dann noch eine Stunde bei Raumtemperatur weiter und filtriert die Lösung zur Abtrennung des Niederschlags. Dieser wird gründlich mit Methanol gewaschen und im Vakuum getrocknet, wobei man 7-Chlor-3,4-dihydro-1-methoxy-3-(4-trifluormethylphenyl)-2(H)-acridon-N-oxid-monohydrat vom Schmelzpunkt 292-94°C erhält.

Unter den oben beschriebenen Bedingungen und ausgehend von einem entsprechend substituierten Acridindion und einem geeigneten, durch Auflösen von Natrium im entsprechenden Alkohol hergestellten Natriumalkoholat werden die folgenden Verbindungen hergestellt:
7-Chlor-3,4-dihydro-1-äthoxy-3-(4-trifluormethylphenyl)-2(H)-acridon-N-oxid-sesquihydrat, Schmelzpunkt > 300°C;
7-Chlor-3,4-dihydro-1-isopropoxy-3-(4-trifluormethylphenyl)-2(H)-acridon-N-oxid-monohydrat, Schmelzpunkt > 300°C;
7-Chlor-3,4-dihydro-1-äthoxy-2-methyl-3-(4-trifluormethylphenyl)-2(H)-acridon-N-oxid, Schmelzpunkt 296°C und
7-Chlor-3,4-dihydro-1-äthoxy-2-phenyl-3-(4-trifluormethylphenyl)-2(H)-acridon-N-oxid, Schmelzpunkt > 300°C.

## Ansprüche

1. Verbindungen der Formel I

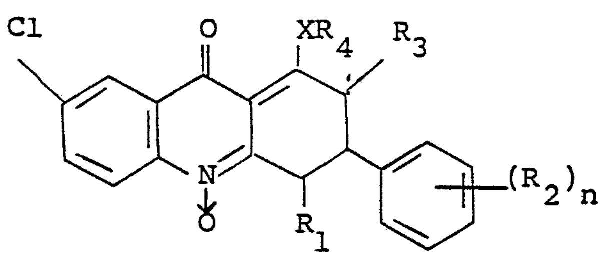

I

worin bedeuten:
$R_1$ und $R_3$, die gleich oder verschieden sein können, Wasserstoff, $C_1$-$C_4$-Alkyl, $C_2$-$C_5$-Carbalkoxy oder Phenyl, welches ein-oder mehrfach mit $C_1$-$C_4$-Alkyl, Halogen oder Amino substituiert sein kann,
$R_2$ Halogen oder Trifluormethyl, wobei die Substituenten falls n eine Zahl von 2 oder 3 bedeutet, gleich oder verschieden sein können,
n eine ganze Zahl von 0 bis 3,
X Sauerstoff oder Stickstoff
$R_4$ falls X Sauerstoff bedeutet, eine $C_1$-$C_4$-Alkylgruppe oder falls X Stickstoff bedeutet,
$XR_4$ eine Di-$C_1$-$C_4$-alkylaminogruppe oder einen 5-oder 6-gliedrigen Heterocyclus, der ein weiteres Heteroatom enthalten kann und unsubstituiert ist oder ein-oder mehrfach substituiert ist mit $C_1$-$C_4$-Alkyl, substituiertem $C_1$-$C_4$-Alkyl, oder Phenyl, das seinerseits ein-oder mehrfach mit $C_1$-$C_4$-Alkyl, $C_1$-$C_4$-Alkoxy oder Halogen substituiert sein kann.

2. Verbindungen der Formel I gemäß Anspruch 1, worin bedeuten:

$R_1$ Wasserstoff

$R_2$ ein Chloratom oder die Trifluormethylgruppe

n die Zahl

$R_3$ Wasserstoff oder Methyl

X ein Sauerstoff-oder ein Stickstoffatom,

$R_4$ falls X Sauerstoff ist, die Methyl-, Äthyl-oder Isopropylgruppe, oder falls X Stickstoff ist,

$XR_4$ einen unsubstituierten oder substituierten Piperazino-, Piperidino-oder Pyrrolidino-Rest.

3. Verfahren zur Herstellung einer Verbindung der Formel I gemäß Anspruch 1, dadurch gekennzeichnet, daß man eine Verbindung der Formel II

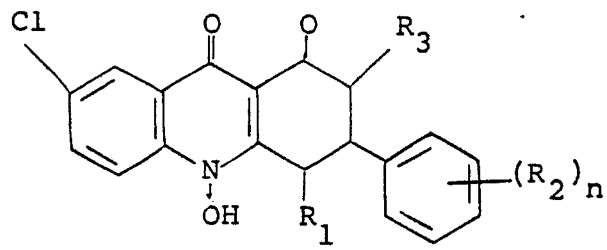

II

worin $R_1$, $R_2$, $R_3$ und n die genannte Bedeutung haben, entweder mit einem Na-Alkoxid der Formel III

$NaOR_4$ III

worin $R_4$ $C_1$-$C_4$-Alkyl bedeutet, zu einer Verbindung der Formel I umsetzt, worin X Sauerstoff darstellt, oder mit einer Verbindung der Formel IV

$HNR_4$ IV

worin $NR_4$ eine Dialkylaminogruppe oder einen 5-oder 6-gliedrigen Heterocyclus darstellt, zu einer Verbindung der Formel I umsetzt, worin X Stickstoff bedeutet und $XR_4$ die genannte Bedeutung hat.

4. Arzneimittel, gekennzeichnet durch einen Gehalt an einer Verbindung der Formel I gemäß Anspruch 1 und pharmazeutisch üblichen Hilfs-und/oder Trägerstoffen.

5. Futterzusatz, gekennzeichnet durch einen Gehalt an einer Verbindung gemäß Anspruch 1 als Wirkstoff.

6. Verwendung einer Verbindung gemäß Anspruch 1 zur Herstellung von Arzneimitteln mit Wirksamkeit gegen Protozoen-Infektionen.

7. Verwendung einer Verbindung gemäß Anspruch 1 zur Herstellung von Arzneimitteln mit Wirksamkeit gegen Malaria.

8. Verwendung einer Verbindung gemäß Anspruch 1 als Futterzusatz zur Verhinderung und Bekämpfung der Coccidiose tei Nutztieren.

Patentansprüche für folgende Vertragsstaaten : ES, AT

1. Verfahren zur Herstellung einer Verbindung der Formel I

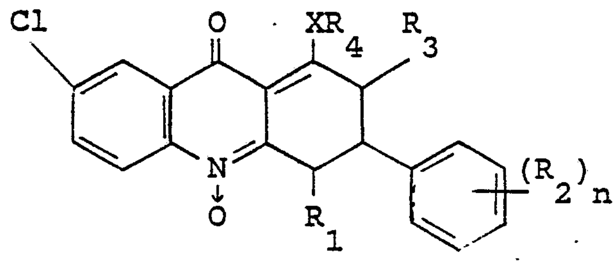

I

worin bedeuten:

$R_1$ und $R_3$, die gleich oder verschieden sein können, Wasserstoff, $C_1$-$C_4$-Alkyl, $C_2$-$C_5$-Carbalkoxy oder Phenyl, welches ein-oder mehrfach mit $C_1$-$C_4$-Alkyl, Halogen oder Amino substituiert sein kann,

$R_2$ Halogen oder Trifluormethyl, wobei die Substituenten falls n eine Zahl von 2 oder 3 bedeutet, gleich oder verschieden sein können,

n eine ganze Zahl von 0 bis 3,

X Sauerstoff oder Stickstoff

$R_4$ falls X Sauerstoff bedeutet, eine $C_2$-$C_4$-Alkylgruppe oder falls X Stickstoff bedeutet,

$XR_4$ eine Di-$C_1$-$C_4$-alkylaminogruppe oder einen 5-oder 6-gliedrigen Heterocyclus, der ein weiteres Heteroatom enthalten kann und unsubstituiert ist oder ein-oder mehrfach substituiert ist mit $C_1$-$C_4$-Alkyl, substituiertem $C_1$-$C_4$-Alkyl, oder Phenyl, das seinerseits ein-oder mehrfach mit $C_1$-$C_4$-Alkyl, $C_1$-$C_4$-Alkoxy oder Halogen substituiert sein kann, dadurch gekenrzeichnet, daß man eine Verbindung der Formel

II

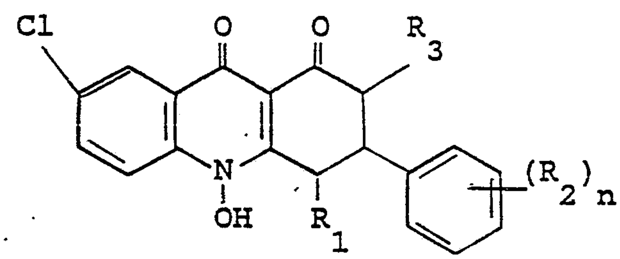

II

worin $R_1$, $R_2$, $R_3$ und n die genannte Bedeutung haben, entweder mit einem Na-Alkoxid der Formel III

$NaOR_4$ III

worin $R_4$ $C_1$-$C_4$-Alkyl bedeutet, zu einer Verbindung der Formel I umsetzt, worin X Sauerstoff darstellt, oder mit einer Verbindung der Formel IV

$HNR_4$ IV

worin $NR_4$ eine Dialkylaminogruppe oder einen 5-oder 6-gliedrigen Heterocyclus darstellt, zu einer Verbindung der Formel I umsetzt, worin X Stickstoff bedeutet und $XR_4$ die genannte Bedeutung hat.

2. Verfahren gemäß Anspruch 1, dadurch gekennzeichnet, daß man eine Verbindung der Formel I herstellt, wobei bedeuten:

$R_1$ Wasserstoff,

$R_2$ Chlor oder die Trifluormethylgruppe,

n die Zahl 1,

$R_3$ Wasserstoff oder Methyl,

X ein Sauerstoff-oder ein Stickstoffatom,

$R_4$, falls X Sauerstoff ist, die Methyl-, Äthyl-oder Isopropylgruppe, oder, falls X Stickstoff ist,

$XR_4$ einen unsubstituierten oder substituierten Piperazino-, Piperidino-oder Pyrrolidino-Rest.

3. Arzneimittel, gekennzeichnet durch einen Gehalt an einer Verbindung der Formel I gemäß Anspruch 1 und pharmazeutisch üblichen Hilfs-und/oder Trägerstoffen.

4. Futterzusatz, gekennzeichnet durch einen Gehalt an einer Verbindung gemäß Anspruch 1 als Wirkstoff.

5. Verwendung einer Verbindung gemäß Anspruch 1 zur Herstellung von Arzneimitteln mit Wirksamkeit gegen Protozoen-Infektionen.

6. Verwendung einer Verbindung gemäß Anspruch 1 zur Herstellung von Arzneimitteln mit Wirksamkeit gegen Malaria.

7. Verwendung einer Verbindung gemäß Anspruch 1 als Futterzusatz zur Verhinderung und Bekämpfung der Coccidiose bei Nutztieren.